# EUROPEAN PATENT APPLICATION

(11) **EP 1 472 992 A1**
(43) Date of publication of application: **03.11.2004**
(21) Application number: 04009952.5
(22) Date of filing: 27.04.2004
(51) Int. Cl.: A61F 2/06

(54) **A method and system for improving stent retention using stent openings**

(30) Priority: 28.04.2003 US 424247
(71) Applicant: Medtronic Vascular, Inc., Santa Rosa, CA 95403 (US)
(72) Inventor: Kantor, John D., Santa Rosa CA 95403 (US)
(74) Representative: Hughes, Andrea Michelle

(57) **Abstract**

The invention provides a system for treating a vascular condition, comprising a catheter 110 and a stent 120. The catheter includes an inflatable balloon 130. The stent is removably coupled to the balloon. The stent includes at least one elongated opening oriented generally perpendicular to the longitudinal axis of the stent. A portion of the balloon is positioned within the elongated opening to aid in retaining the stent to the balloon.

## Description

### TECHNICAL FIELD

This invention relates generally to biomedical devices that are used for treating vascular conditions. More specifically, the invention relates to a system and method for improving stent retention using at least one elongated stent opening oriented generally perpendicular to the longitudinal axis of the stent and a portion of a balloon positioned within the elongated opening.

### BACKGROUND OF THE INVENTION

Stents are generally cylindrical-shaped devices that are radially expandable to hold open a segment of a vessel or other anatomical lumen after implantation into the body lumen. Various types of stents are in use, including expandable and self-expanding stents. Expandable stents generally are conveyed to the area to be treated on balloon catheters or other expandable devices. For insertion, the stent is positioned in a compressed configuration along the delivery device, for example crimped onto a balloon that is folded or otherwise wrapped about a guide wire lumen that is part of the delivery device. After the stent is positioned across the lesion, it is expanded by the delivery device, causing the diameter of the stent to expand. For a self-expanding stent, commonly a sheath is retracted, allowing expansion of the stent.

Stents are used in conjunction with balloon catheters in a variety of medical therapeutic applications, including intravascular angioplasty. For example, a balloon catheter device is inflated during percutaneous transluminal coronary angioplasty (PTCA) to dilate a stenotic blood vessel. The stenosis may be the result of a lesion such as a plaque or thrombus. When inflated, the pressurized balloon exerts a compressive force on the lesion, thereby increasing the inner diameter of the affected vessel. The increased interior vessel diameter facilitates improved blood flow. Soon after the procedure, however, a significant proportion of treated vessels restenose.

To prevent restenosis, a stent, constructed of a metal or polymer, is implanted within the vessel to maintain lumen size. The stent acts as a scaffold to support the lumen in an open position. Configurations of stents include a cylindrical tube defined by a mesh, interconnected stents, or like segments. Exemplary stents are disclosed in U.S. Patent No. 5,292,331 to Boneau, U.S. Patent No. 6,090,127 to Globerman, U.S. Patent No. 5,133,732 to Wiktor, U.S. Patent No. 4,739,762 to Palmaz, and U.S. Patent No. 5,421,955 to Lau.

For a stent to provide the desired beneficial effect, it must be delivered to precisely the correct position within a vessel. Prior art stent delivery systems have encountered difficulty maintaining the stent on the delivery catheter while positioning the stent within the vessel. Stents have been dislodged and lost while being delivered to a lesion, while being deployed at the treatment site, or while being retracted from the body following an unsuccessful treatment of the lesion site. Therefore, it would be desirable to provide a method and system for retaining a stent to a catheter for delivery and deployment of the stent in a vessel that overcomes the aforementioned and other disadvantages.

### SUMMARY OF THE INVENTION

One aspect of the present invention is a system for treating a vascular condition, comprising a catheter and a stent. The catheter includes an inflatable balloon. The stent is removably coupled to the balloon. The stent includes at least one elongated opening oriented generally perpendicular to the longitudinal axis of the stent. A portion of the balloon is positioned within the elongated opening to aid in retaining the stent to the catheter.

Another aspect of the present invention is a method of making a system for treating a vascular condition. A catheter is provided, the catheter including an inflatable balloon. A stent is provided, the stent including at least one elongated opening oriented generally perpendicular to the longitudinal axis of the stent. The stent is placed over the balloon such that a portion of the balloon is positioned within the elongated opening, wherein the balloon portion aids in retaining the stent to the catheter.

The aforementioned and other features and advantages of the invention will become further apparent from the following detailed description of the presently preferred embodiments, read in conjunction with the accompanying drawings. The detailed description and drawings are merely illustrative of the invention rather than limiting, the scope of the invention being defined by the appended claims.

### BRIEF DESCRIPTION OF THE DRAWINGS

**FIG. 1** is an illustration of one embodiment of a system for treating a vascular condition, in accordance with the present invention;
**FIG. 2** is an enlarged, fragmentary view of the stent shown in **FIG. 1;**
**FIG. 3** is an enlarged, fragmentary view of another embodiment of a stent in accordance with the present invention; and
**FIG. 4** is a flow diagram of one embodiment of a method of making a system for treating a vascular condition, in accordance with the present invention.

### DETAILED DESCRIPTION OF THE PRESENTLY PREFERRED EMBODIMENTS

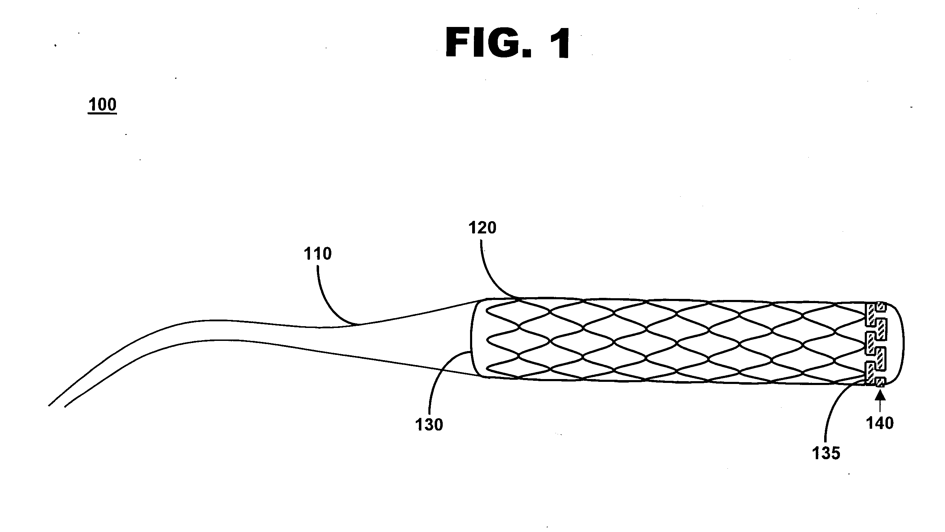

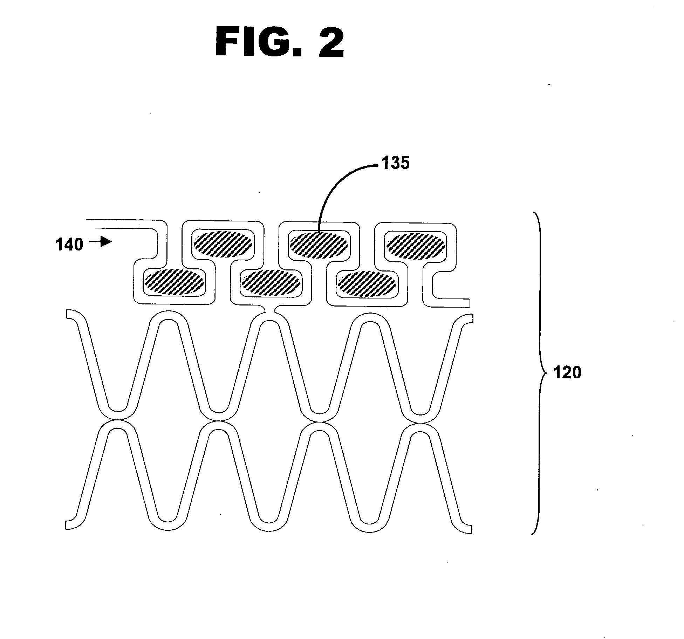

One aspect of the present invention is a system for treating a vascular condition. One embodiment of the system, in accordance with the present invention, is illustrated in **FIG. 1** at **100**. System **100** comprises a catheter **110** and a stent **120**. Catheter **110** includes an inflatable balloon **130**. Stent **120**, includes a plurality of elongated openings **140** oriented generally perpendicular to the longitudinal axis of the stent. Balloon portions **135** are positioned within the elongated openings **140** to aid in retaining stent **120** to catheter **110**. **FIG. 2** shows an enlarged, fragmentary view of stent **120**, in which like elements share like reference numbers with **FIG. 1**.

Catheter **110** may be any catheter known in the art that is appropriate for delivering a stent to a treatment site within a vessel, for example a percutaneous transluminal coronary angioplasty (PTCA) balloon catheter. Inflatable balloon **130** may be made of a suitable material such as polyethylene, polyethylene terephthalate (PET), or from nylon or the like. The length and diameter of balloon 130 may be selected based on the dimensions of the stent being delivered.

Stent **120** is removably coupled to balloon **130** and thereby to catheter **110.** Stent **120** may be made of a wide variety of medical implantable materials, including, but not limited to, stainless steel, nitinol, tantalum, ceramic, nickel, titanium, aluminum, polymeric materials, MP35N, stainless steel, titanium ASTM F63-83 Grade 1, niobium, high carat gold K 19-22, and combinations thereof.

A therapeutic coating (not shown) may be disposed on at least a portion of the stent. The therapeutic coating may include, for example, an antineoplastic agent, an antiproliferative agent, an antibiotic, an antithrombogenic agent, an anticoagulant, an antiplatelet agent, an anti-inflammatory agent, combinations of the above, and the like.

Stent **120** includes elongated openings **140** oriented generally perpendicular to the longitudinal axis of the stent. These openings may be, for example, laser cut into a stent wall in the case of a tubular stent, formed by bending a ring, or formed by bending into the segments of a wire coil stent.

Balloon portions **135** are positioned within these openings. The balloon portions may be positioned within the elongated openings in response to, for example, pressurizing the balloon, heating the balloon, or combinations thereof. Balloon portions **135**, like the openings **140** in which they are formed, are oriented generally perpendicular to the longitudinal axis of the stent. They can, therefore, provide greater resistance to stent slippage, displacement, or dislodgment than can structures formed within the openings of a standard modular stent, for example, which has elongated openings oriented parallel to the longitudinal axis of the stent. Balloon portions **135** of the present invention thus aid in retaining stent **120** to catheter **110.**

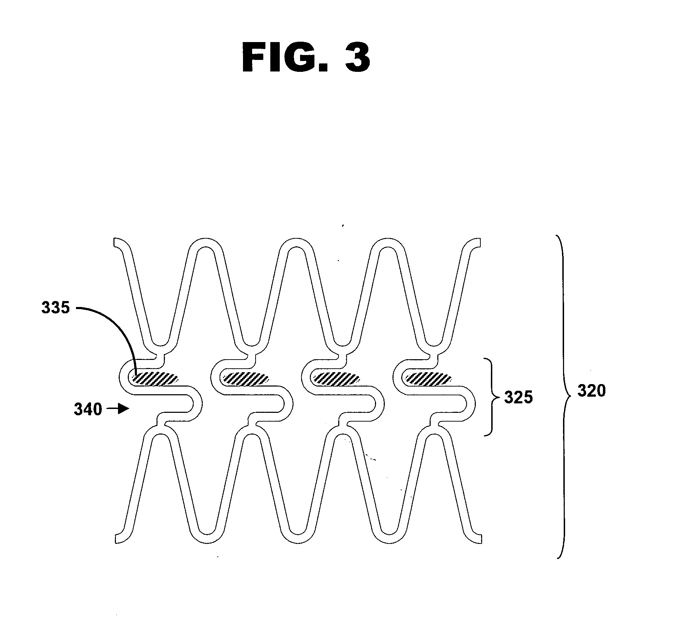

FIG. 3 is an enlarged, fragmentary view of another embodiment of a stent in accordance with the present invention. Balloon portions **335** are shown positioned within the elongated openings **340** formed by the stent segment connectors **325** of stent **320.** In **FIGS. 1** and **2,** the elongated openings are shown positioned on one end portion of the stent. However, elongated openings may be positioned on both end portions of a stent or throughout the stent, as with the stent segment connectors shown in **FIG. 3**. One skilled in the art will recognize that the stents shown in **FIGS. 1, 2**, and **3** are just two possible embodiments of the present invention and the elongated openings may assume a variety of shapes and sizes cut or formed into the stent.

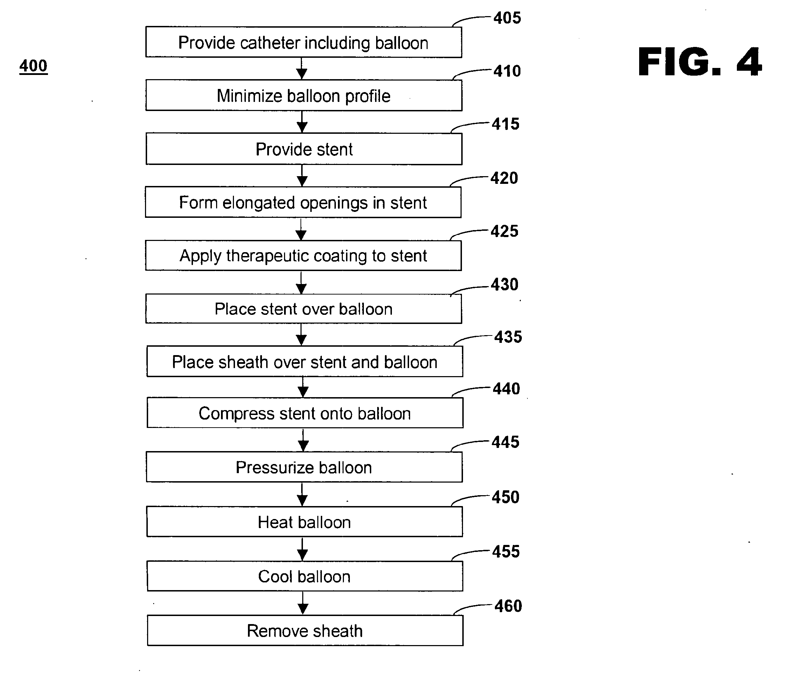

Another aspect of the present invention is a method of making a system for treating a vascular condition. **FIG. 4** shows a flow diagram of one embodiment in accordance with the present invention at**400**.

A catheter is provided **(Block 405),** the catheter including an inflatable balloon. The catheter may be any catheter known in the art that is appropriate for delivering a stent to a lesion site identified for treatment, for example a percutaneous transluminal coronary angioplasty (PTCA) balloon catheter. The balloon may be made from a suitable material such as polyethylene, polyethylene terephthalate (PET), or from nylon or the like. The length and diameter of the balloon may be selected based on the dimensions of the stent being delivered. The balloon is folded or otherwise manipulated or treated to minimize its profile (**Block 410**).

A stent is provided (**Block 415**). The stent includes at least one elongated opening oriented generally perpendicular to the longitudinal axis of the stent. Providing such a stent may comprise, for example, creating a plurality of elongated openings in the stent by cutting the openings into the wall of the stent, forming the openings into a ring, or forming the openings into the segments of a wire coil stent (**Block 420**). A therapeutic coating may be applied to the stent by, for example, spraying or dipping the stent so that the coating is over the outer surface of the stent (**Block 425**).

The stent is placed over the balloon (**Block 430**). In this embodiment, placing the stent over the balloon comprises the following steps. Once the stent is in position over the balloon, a sheath made of a material such as polytetrafluoroethylene (PTFE) is positioned over the stent (**Block 435**), thereby enclosing both the stent and the balloon. The interior diameter of the sheath may aid in defining the shape and size of the balloon portions that are positioned within the elongated openings. The sheath may also protect the stent from damage when it is compressed onto the balloon (**Block 440**).

The balloon is pressurized with an inflation pressure within the range of, for example, one hundred fifty to two hundred pounds per square inch (150-200 PSI) to position portions of the balloon within the elongated openings (**Block 445**). The balloon is then heated to heat-set the balloon material, thus maintaining the balloon portions within the elongated openings (**Block 450**). This may be accomplished by, for example, heating the balloon and attached stent in a heat set block at a temperature within the range of one hundred fifty to one hundred eighty degrees Fahrenheit (150-180°F) for two to five minutes. Alternatively, portions of the balloon may be positioned within the elongated openings by heating without pressurizing, relying on expansion of the balloon material to position the portions within the openings.

The system may be actively cooled, or it may be allowed to cool at room temperature (**Block 455**). Once the assembly has cooled, the sheath may be removed from the assembly (**Block 460**). The sheath may also be left in place to protect the balloon and stent during shipping or storage.

The resulting balloon structures, like the openings in which they are formed, are oriented generally perpendicular to the longitudinal axis of the stent. They can, therefore, provide greater resistance to stent slippage, displacement, or dislodgment than can structures formed within the openings of a standard modular stent, for example, which has elongated openings oriented parallel to the longitudinal axis of the stent. The present invention thus improves retention of a stent on a delivery catheter.

## Claims

1. A system for treating a vascular condition, comprising:
a catheter (110), the catheter including an inflatable balloon (130); and
a stent (120) removably coupled to the balloon, the stent including at least one elongated opening (140) oriented generally perpendicular to the longitudinal axis of the stent, wherein a portion of the balloon is positioned within the elongated opening to aid in retaining the stent to the catheter.

2. The system of claim 1 wherein the elongated opening is cut into a stent wall.

3. The system of claim 1 wherein the elongated opening is formed into a stent segment.

4. The system of any preceding claims, wherein the portion of the balloon is positioned within the elongated opening in response to an action selected from a group consisting of inflating the balloon, heating the balloon, and combinations thereof.

5. The system of any preceding claim, wherein the stent includes a plurality of openings oriented generally perpendicular to the longitudinal axis of the stent.

6. The system of claim 5 wherein the openings are positioned on at least an end portion of the stent.

7. The system of claim 5 wherein the openings are positioned throughout the stent.

8. The system of any preceding claim further comprising:
a therapeutic coating disposed on at least a portion of the stent.

9. The system of claim 8 wherein the therapeutic coating includes a therapeutic agent selected from a group consisting of an antineoplastic agent, an antiproliferative agent, an antibiotic, an antithrobogenic agent, and anticoagulant, an antiplatelet agent, an anti-inflammatory agent, and combinations thereof.

10. A method of making a system for treating a vascular condition, comprising:
providing a catheter (110), the catheter including an inflatable balloon (130);
providing a stent (120), the stent including at least one elongated opening oriented generally perpendicular to the longitudinal axis of the stent; and
placing the stent over the balloon such that a portion of the balloon is positioned within the elongated opening, wherein the balloon portion aids in retaining the stent to the catheter.

11. The method of claim 10 further comprising:
minimizing the balloon profile prior to placing the stent over the balloon.

12. The method of claim 10 or 11, further comprising:
applying a therapeutic agent coating to at least a portion of the stent.

13. The method of claim 10, 11 or 12, wherein providing the stent comprises cutting at least one elongated opening into a wall of the stent.

14. The method of claim 10, 11 or 12, wherein providing the stent comprises forming at least one elongated opening into a segment of the stent.

15. The method of any of claims 10 to 14, wherein placing the stent over a balloon comprises compressing the stent onto the balloon.

16. The method of any of claims 10 to 14 wherein placing the stent over a balloon comprises pressurizing the balloon to position a portion of the balloon within the elongated opening.

17. The method of any of claims 10 to 14, wherein placing the stent over a balloon comprises heating the balloon to position a portion of the balloon,within the elongated opening.

18. The method of claim 16 further comprising:
heating the balloon to maintain a portion of the balloon within the elongated opening.

19. The method of claim 16 further comprising:
positioning a sheath over the stent and the balloon prior to pressurizing the balloon.

20. The method of claim 17 further comprising:
positioning a sheath over the stent and the balloon prior to heating the balloon.
